# EUROPEAN PATENT SPECIFICATION

(11) **EP 0 626 169 B1**
(45) Date of publication and mention of the grant of the patent: **14.07.1999**
(21) Application number: 94201844.1
(22) Date of filing: 25.08.1989
(51) Int. Cl.: A61K 39/145, A61K 9/127, A61K 39/39

(54) **A dosage form comprising an antigen and a salt form of an organic acid derivative of a sterol**
Dosierungsform die ein Antigen und eine Salzform eines organischen Säurederivats eines Sterols enthält
Forme de dosage comprenant un antigène et un sel d'un dérivé organoacide d'un stérol

(30) Priority: 25.08.1988 US 236701; 25.08.1988 US 236702; 23.08.1989 US 397777
(43) Date of publication of application: 30.11.1994
(62) Divisional of application: 89402343.1
(73) Proprietor: THE LIPOSOME COMPANY, INC., Princeton, NJ 08540 (US)
(72) Inventor: Popescu, Mircea C., Plainsboro, New Jersey 08536 (US); Recine, Marie S., Hamilton Twp., New Jersey 08690 (US); Alving, Carl L., Bethesday, Maryland 20817 (US); Estis, Leonard F., Upton, Massachusetts 01568 (US); Keyes, Lynn D., Upton, Massachusetts 01568 (US); Janoff, Andrew S., Yardley, Pennsylvania 19067 (US)
(74) Representative: Warcoin, Jacques

(56) References cited:
- EP-A- 0 011 549
- WO-A-85/04578
- WO-A-86/05977
- FR-A- 2 276 062
- VACCINE, vol.5, June 1987 pages 145 - 150 G. GREGORIADIS ET AL. 'Liposomes as immunological adjuvants:antigen incorporation studies'
- THE LANCET, 8 November 1975 pages 899 - 901 J.D. ALMEIDA ET AL. 'Formation of virosomes from influenza subunits and liposomes'
- CHEMICAL ABSTRACTS, vol. 98 Columbus, Ohio, US; abstract no. 137302, MEIR , SHINITZKY ET AL. 'Regulation of tumor growth by lipids. Possible clinical applications' & DEV. CANCER RES. (1982), vol.7 pages 61 - 68 '(Membr. Tumour Growth)'

## Description

### Field of the Invention

This invention in the vaccine arts is concerned with an influenza immunizing dosage form comprising a liposome and an antigen of Influenza, particularly the hemagglutinin or bromelain fragment, wherein said liposome and antigen are present in an immunization dose. Additionally, a dosage form, including such form particularly adapted to producing an immune response, comprising a salt form of an organic acid derivative of a sterol and an antigen wherein said organic acid derivative of a sterol and antigen are present in an immunization dose, and method of use. Further, a dosage form, including such form particularly adapted to producing an immune response, comprising dimyristolyphosphatidylcholine (DMPC)/cholesterol liposomes, optionally in an aluminum hydroxide gel, and an antigen wherein said DMPC/cholesterol and antigen are present in an immunization dose, and method of use.

### Background of the Invention

In the vaccine art antigens are introduced into an organism in a manner so as to stimulate an immune response in the host organism. The induction of an immune response depends on many factors among which are believed to include the chemical composition and configuration of the antigen, the immunogenic constitution of the challenged organism, and the manner and period of administration of the antigen. An immune response has many facets some of which are exhibited by the cells of the immune system, (e.g.,B-lymphocytes, T-lymphocytes, macrophages, and plasma cells). Immune system cells may participate in the immune response through interaction with antigen, interaction with other cells of the immune system, the release of cytokines and reactivity to those cytokines. Immune response is conveniently (but arbitrarily) divided into two main categories -- humoral and cell-mediated. The humoral component of the immune response includes production of immunoglobulins specific for the antigen. The cell-mediated component includes the generation of delayed-type hypersensitivity and cytotoxic effector cells against the antigen.

In some instances immune response is the result of an initial or priming dose of an antigen that is followed by one or more booster exposures to the antigen. Priming with relatively strong immunogens and liposomes is discussed in "Liposomal Enhancement of the Immunogenicity of Adenovirus Type 5 Hexon and Fiber Vaccines", Kramp, W.J. et al., Infection and Immunity, 25:771-773 (1979) and "Liposomes as Adjuvants with Immunopurified Tetanus Toxoid: the Immune Response", Davis, D. et al., Immunology Letters, 14:341-8 (1986/1987).

Ideally, an antigen will exhibit two properties, the capacity to stimulate the formation of the corresponding antibodies and the propensity to react specifically with these antibodies. Immunogens bear one or more epitopes which are the smallest part of an antigen recognizable by the combining site of an antibody or immunoglobulin.

In particular instances antigens or fractions of antigens or with particular presenting conditions the immune response precipitated by the desired antigen is inadequate or nonexistent and insufficient immunity is produced. This is particularly the case with peptide or other small molecules used as immunogens.

In such cases the vaccine art recognizes the use of substances called adjuvants to potentiate an immune response when used in conjunction with an antigen. Adjuvants are further used to elicit immune response sooner, or a greater response, or with less antigen or to increase production of certain antibody subclasses that afford immunological protection, or to enhance components of the immune response (e.g., humoral, cellular).

Well known adjuvants are Freund's Adjuvants (and other oil emulsions), Bortedella Pertussis, aluminum salts (and other metal salts), Mycobacterial products (including muramyl dipeptides), and liposomes. As used herein the term "adjuvant" will be understood to mean a substance or material administered together or in conjunction with an antigen which increases the immune response to that antigen. Adjuvants may be in a number of forms including emulsion (e.g., Freund's adjuvant) gel, (aluminum hydroxide gel) and particles (liposomes) or as a solid material. Liposomal vaccines and adjuvancy are further discussed in WO 90 01 947 herewith the teachings of which are incorporated herein by reference.

It is believed that adjuvant activity can be effected by a number of factors. Among such factors are (a) carrier effect, (b) depot formation, (c) altered lymphocyte recirculation, (d) stimulation of T-lymphocytes, (e) direct stimulation of B-lymphocytes and (f) stimulation of macrophages.

With many adjuvants adverse reactions are seen. In some instances adverse reactions include granuloma formation at the site of injection, severe inflammation at the site of injection, pyrogenicity, adjuvant induced arthritis or other autoimmune response, or oncogenic response. Such reactions have hampered the use of adjuvants such as Freund's adjuvant.

In particular embodiments adjuvants are comprised of liposomes. U.S. Patent No. 4,053,585 issued October 17, 1977 to Allison et al. states that liposomes of a particular charge are adjuvants. Davis, D, et al., "Liposomes as Adjuvants with Immunopurified Tetanus Toxoid: Influence of Liposomal Characteristics", Immunology, 61: 229-234 (1987) and; Gregoriadis, G. et al., "Liposomes as Immunological Adjuvants: Antigen Incorporation Studies", Vaccine, 5:145-151 (1987) report DMPC/cholesterol liposomes (1:1) and antigen as giving minimally improved (over free antigen) immunological , response in small unilamellar vesicles of a distinct dehydration/rehydration type with tetanus toxoid as the antigen, a strong immunogen. In the Davis and in the Gregoriadis papers, the liposomal immunogenic response was only minimally distinguishable from the response of free antigen. To distinguish the liposomal from free antigen response it was necessary for the authors to dilute the tetanus toxoid to minimal response amounts. The present invention adopts conditions of DMPC/cholesterol liposomes that yield a therapeutically effective immunological response.

Other substances such as immunomodulators (e.g., cytokines such as the interleukins) may be combined in adjuvants/vaccines as well.

Humoral immune response may be measured by many well known methods. Single Radial Immunodifussion Assay (SRID), Enzyme Immunoassay (EIA) and Hemagglutination Inhibition Assay (HAI) are but a few of the commonly used assays of humoral immune response.

SRID utilizes a layer of a gel such as agarose containing the immunogen being tested. A well is cut in the gel and the serum being tested is placed in the well. Diffusion of the antibody out into the gel leads to the formation of a precipitation ring whose area is proportional to the concentration of the antibody in the serum being tested.

EIA, also known as ELISA (Enzyme Linked Immunoassay), is used to determine total antibodies in a sample. The antigen is adsorbed to the surface of a microtiter plate. The test serum is exposed to the plate followed by an enzyme linked immunogloublin, such as IgG. The enzyme activity adherent to the plate is quantified by any convenient means such as spectrophotometry and is proportional to the concentration of antibody directed against the antigen present in the test sample.

HAI utilizes the capability of an antigen such as viral proteins to agglutinate chicken red blood cells (or the like). The assay detects neutralizing antibodies, i.e. those antibodies able to inhibit hemagglutination. Dilutions of the test serum are incubated with a standard concentration of antigen, followed by the addition of the red blood cells. The presence of neutralizing antibodies will inhibit the agglutination of the red blood cells by the antigen.

Tests to measure allergic and cellular immune response include determination of delayed-type hypersensitivity or measuring the proliferative response of lymphocytes to target antigen.

Liposomes are completely closed lipid bilayer membranes containing an entrapped aqueous volume. Liposomes may be unilamellar vesicles (possessing a single bilayer membrane ) or multilameller vesicles (onion-like structures characterized by multiple membrane bilayers, each separated from the next by an aqueous layer). The bilayer is composed of two lipid monolayers having a hydrophobic "tail" region and a hydrophilic "head" region. The structure of the membrane bilayer is such that the hydrophobic (nonpolar) "tails" of the lipid monolayers orient toward the center of the bilayer while the hydrophilic "head" orient towards the aqueous phase.

The original liposome preparation of Bangham, et al. (J. Mol. Biol., 1965, 13:238-252) involves suspending phospholipids in an organic solvent which is then evaporated to dryness leaving a phospholipid film on the reaction vessel. Next, an appropriate amount of aqueous phase is added, the mixture is allowed to "swell," and the resulting liposomes which consist of multilamellar vesicles (MLVs) are dispersed by mechanical means. This technique provides the basis for the development of the small sonicated unilamellar vesicles described by Papahadjopoulos et al. (Biochim. Biophys. Acta., 1968, 135:624-638), and large unilamellar vesicles. Small unilamellar vesicles have a diameter of about 100nm or less.

Unilamellar vesicles may be produced using an extrusion apparatus by a method described in Cullis et al., PCT Application No. WO 87/00238, published January 16, 1986, entitled "Extrusion Technique for Producing Unilamellar Vesicles" incorporated herein by reference. Vesicles made by this technique, called LUVETS, are extruded under pressure once or a number of times through a membrane filter. LUVETs will be understood to be included in the term "unilamellar vesicle".

Another class of multilamellar liposomes are those characterized as having substantially equal lamellar solute distribution. This class of liposomes is denominated as stable plurilamellar vesicles (SPLV) as defined in U.S. Patent No. 4,522,803 to Lenk, et al., monophasic vesicles as described in U.S. Patent No. 4,588,578 to Fountain, et al. and frozen and thawed multilamellar vesicles (FATMLV) wherein the vesicles are exposed to at least one freeze and thaw cycle; this procedure is described in Bally et al., PCT Publication No. 87/00043, January 15, 1987, entitled "Multilamellar Liposomes Having Improved Trapping Efficiencies". U.S. Patent No. 4,721,612 to Janoff et al. describes steroidal liposomes for a variety of uses. The teachings of these references as to preparation and use of liposomes are incorporated herein by reference.

### Summary of the Invention

In one aspect this invention includes an influenza immunizing dosage form comprising a liposome and an antigen of Influenza wherein said liposome and antigen are present in an immunization dose. In a particular embodiment the antigen comprises the hemagglutinin fragment or the bromelain fragment. In an additional embodiment the liposome comprises a salt form of an organic acid derivative of a sterol. In a given dosage form the antigen is entrapped in the liposome, preferably, a multilamellar vesicle, and further preferably at least about 1 micron in diameter. A particularly useful liposome comprises a tris (hydroxymethyl) aminomethane salt form of an organic acid derivative of a sterol. Another particularly useful liposome comprises DMPC/cholesterol with particular reference to a mole ratio of from about 80 to about 20 DMPC to from about 20 to about 80 cholesterol, and more particularly wherein said ratio is from about 40:60 to about 60:40 and further wherein said liposome is a multilamellar vesicle such as one of substantially equal lamellar solute distribution (SPLV).

This invention includes a dosage form comprising a salt form of an organic acid derivative of a sterol and an antigen wherein said organic acid derivative of a sterol and an antigen are present in an immunization dose. In one embodiment the dosage form is a liposome such as a multilamellar vesicle, particularly those multilamellar vesicles at least about 1 micron in diameter. In some embodiments the antigen is entrapped in the liposome.

In particular embodiments the dosage form, the salt form of the organic acid derivative of a sterol is a tris (hydroxymethyl) aminomethane. In other embodiments the salt form is a carboxylic acid derivative of a sterol (such as an aliphatic carboxylic acid, particularly those up to five carbon atoms), a salt form of a dicarboxylic acid derivative of a sterol (such as an aliphatic dicarboxylic acid, particularly those up to seven carbon atoms), an hydroxy acid derivative of a sterol (such as citric acid), an amino acid derivative of a sterol or a salt form of a polyamino acid derivative of a sterol, or a salt form of a polycarboxylic acid derivative of a sterol.

In one embodiment of the dosage form the aliphatic dicarboxylic acid is succinate.

In specific embodiments of the dosage form of this invention the immunogen is selected from the group comprising proteins, peptides, polysaccharides, nucleic acids, lipids, glycolipids, lipoproteins, lipopolysaccharides, synthetic peptides or bacterial fractions, viral fractions, protozoal fractions, tissue fractions, or cellular fractions. Specific antigens are influenza fractions such as hemagglutinin, parainfluenza 3 (fusion and hemagglutinin-neuraminidase), malaria sporozoite fractions, hepatitis (A, B, and non-A/non-B) fractions, meningococcus fractions, HIV fractions (all strains), and melanoma fractions.

The dosage form of the invention may further include an immunomodulator such as a cytokine (e.g., interferons, thrombocytic derived factors, monokines and lymphokines such as IL2).

Another aspect of this invention is a composition for potentiating an immune response in an animal, including a human, comprising an immunization dose of a composition comprising an organic acid derivative of a sterol and an antigen. In one embodiment the composition for potentiating the immune response includes a liposome such as a multilamellar vesicle, particularly those multilamellar vesicles at least about 1 micron in diameter. In some embodiments the antigen is entrapped in the liposome.

In particular embodiments this composition for potentiating immune response includes the salt form of organic acid derivative of a sterol being a tris (hydroxymethyl) aminomethane. In other embodiments the salt form is a carboxylic acid derivative of a sterol (such as an aliphatic carboxylic acid, particularly those up to five carbon atoms), a salt form of a dicarboxylic acid derivative of a sterol (such as an aliphatic dicarboxylic acid, particularly those up to seven carbon atoms), an hydroxy acid derivative of a sterol (such as citric acid), an amino acid derivative of a sterol or a salt form of a polyamino acid derivative of a sterol, or a salt form of a polycarboxylic acid derivative of a sterol.

In one embodiment of the composition for potentiating an immune response the aliphatic dicarboxylic acid is succinate.

In specific embodiments of the composition for potentiating an immune response of this invention the antigen is selected from the group comprising proteins, peptides, polysaccharides, nucleic acids, lipids, glycolipids, lipoproteins, lipopolysaccharides, synthetic peptides or bacterial fractions, viral fractions, protozoal fractions, tissue fractions, or cellular fractions.

The composition for potentiating an immune response of the invention may further include using an immunomodulator such as a cytokine.

Another embodiment of the invention comprises a composition for potentiating an immune response in an animal, including a human, comprising the use of an adjuvant wherein the adjuvant comprises a salt form of an organic acid derivative of a sterol.

In particular embodiments this composition for potentiating immune response by use of an adjuvant includes the salt form of an organic acid derivative of a sterol being a tris (hydroxymethyl) aminomethane or a sodium salt. In other embodiments the salt form is a carboxylic acid derivative of a sterol (such as an aliphatic carboxylic acid, particularly those up to five carbon atoms), a salt form of a dicarboxylic acid derivative of a sterol (such as an aliphatic dicarboxylic acid, particularly those up to seven carbon atoms), an hydroxy acid derivative of a sterol (such as citric acid), an amino acid derivative of a sterol or a salt form of a polyamino acid derivative of a sterol, or a salt form of a polycarboxylic acid derivative of a sterol.

In one embodiment of the composition for potentiating an immune response the aliphatic dicarboxylic acid is succinate.

This invention yet further comprises a composition for priming an immune response in an animal, including a human, comprising a priming immunization dose of a composition comprising a liposome adjuvant -- any type of liposome -- and particularly a liposome which is an organic acid derivative of a sterol and an adjuvant-obligatory immunogen such that administration of a booster dose of adjuvant-obligatory immunogen absent adjuvant further potentiates immune response. The composition for priming an immune response in an animal, in a particular embodiment, comprises a salt form of an organic acid derivative of a sterol wherein the salt form is a tris (hydroxymethyl) aminomethane or sodium salt form of an organic acid derivative of a sterol. Priming, using an immunogen that would not generate an immune response absent an adjuvant is particularly included in this embodiment.

In other embodiments of the composition for priming an immune response in an animal, the salt form is a carboxylic acid derivative of a sterol (such as an aliphatic carboxylic acid, particularly those up to five carbon atoms), a salt form of a dicarboxylic acid derivative of a sterol (such as an aliphatic dicarboxylic acid, particularly those up to seven carbon atoms), an hydroxy acid derivative of a sterol (such as citric acid), an amino acid derivative of a sterol or a salt form of a polyamino acid derivative of a sterol, or a salt form of a polycarboxylic acid derivative of a sterol. In addition SPLV liposomes, or multilamellar liposomes, especially those of about 1 micron or more are used as well as liposomes that comprises phosphatidylcholine, cholesterol, phosphatidyl serine or phosphatidyl ethanolamine. A preferred embodiment of a method of priming further includes immunizing a primed animal by the step of administering to said animal at least one booster dose of adjuvant-obligatory immunogen absent adjuvant.

A method of conferring immunity on an animal, including a human, comprises the step of administering to such animal a therapeutically effective immunization course at least one element of which is administering an immunization dose of a composition comprising an antigen and an organic acid derivative of a sterol. In one embodiment the composition further comprises a liposome (including multilamellar vesicles) and preferably wherein the antigen is entrapped in the liposome as well as liposomes at least about 1 micron in diameter. In employing this method the composition further can comprise a tris (hydroxymethyl) aminomethane salt form of an organic acid derivative of a sterol, or a salt form of a carboxylic acid derivative of a sterol such as an aliphatic carboxylic acid (optionally up to 5 carbon atoms), a salt form of a dicarboxylic acid derivative of a sterol such as an aliphatic dicarboxylic acid optionally up to seven carbon atoms (e,g, succinate), or a salt form of a polycarboxylic acid derivative of a sterol. In particular embodiments of the method the composition further comprises a salt form of an hydroxy acid derivative of a sterol such as citric acid.

Further, this invention includes a dosage form comprising an immunogen and a multilamellar liposome comprising DMPC/cholesterol in an immunization dose, in one embodiment further including aluminum adjuvants such as aluminum hydroxide gel. In one embodiment the liposome of the dosage form comprises a mole ratio of from about 80 to about 20 DMPC to from about 20 to about 80 cholesterol, and particularly wherein the ratio is from about 30:70 to about 70:30 and preferably 70:30. In specific embodiments the dosage form multilamellar liposome is of equal solute distribution (SPLV) and/or at least 1 micron in diameter and particularly a 70:30 mole ratio DMPC/cholesterol SPLV.

In particular embodiments of the dosage form the antigen is selected from the group comprising proteins, peptides, polysaccharides, bacterial fractions, viral fractions, protozoal fractions, synthetic peptides or lipopolysaccharides. Furthermore the dosage form may comprise an immunomodulator including a cytokine. Additionally the dosage form may comprise a suitable pharmaceutical carrier.
Another aspect of this invention includes a composition for potentiating an immune response in an animal, including a human, comprising an immunization dose of a composition comprising an antigen and a multilamellar liposome comprising DMPC/cholesterol, and optionally further including aluminum adjuvants such as aluminum hydroxide gel. In one embodiment the liposomes comprise a mole ratio of from about 80 to about 20 DMPC to from about 20 to about 80 cholesterol, and particularly wherein the ratio is from about 30:70 to about 70:30. In specific embodiments of the method the multilamellar liposome is of equal solute distribution (e.g., SPLV) and/or at least about 1 micron in diameter and particularly a 70:30 mole ratio DMPC/cholesterol SPLV.
In particular embodiments the antigen is selected from the group comprising proteins, peptides, polysaccharides, bacterial fractions, viral fractions, protozoal fractions, synthetic peptides or lipopolysaccharides. Furthermore in employing the method the dose may comprise an immunomodulator including a cytokine. Additionally the dosage form may comprise a suitable pharmaceutical carrier.
Additionally included in this invention is a composition for potentiating an immune response in an animal including a human comprising the use of an adjuvant wherein the adjuvant comprises a liposome comprising DMPC/cholesterol, in one embodiment further including aluminum adjuvants such as aluminum hydroxide gel. In one embodiment the liposomes comprise a mole ratio of from about 80 to about 20 DMPC to from about 20 to about 80 cholesterol, and particularly wherein the ratio is from about 30:70 to about 70:30. In specific embodiments the multilamellar liposome is an SPLV and/or at least about 1 micron in diameter and particularly a 70:30 DMPC/cholesterol SPLV.

In particular embodiments, the antigen is selected from the group comprising proteins, peptides, polysaccharides, bacterial fractions, viral fractions, protozoal fractions, synthetic peptides and lipopolysaccharides. Furthermore in employing the method the dose may comprise an immunomodulator including a cytokine. Additionally the dose form may comprise a suitable pharmaceutical carrier.

A further embodiment of the invention is a composition for priming an immune response in an animal, including a human, comprising a priming immunization dose of a composition comprising an adjuvant which is a multilamellar liposome comprising DMPC/cholesterol and an adjuvant-obligatory immunogen (and optionally aluminum adjuvants such as aluminum hydroxide gel) such that administration of a booster dose of adjuvant-obligatory immunogen absent adjuvant further potentiates immune response, The composition for priming, in specific instances includes the liposome being an SPLV multilamellar vesicle and/or the liposome being at least about 1 micron in diameter, and preferably about 70:30 DMPC/cholesterol. Specific immunogens of the dosage form and the methods are influenza fractions such as hemagglutinin, parainfluenza 3 (fusion and hemagglutinin-neuraminidase), malaria sporozoite fractions, hepatitis (A, B, and non-A/non-B) fraction, meningococcus fractions, HIV fractions (all strains), and melanoma fractions. A method of conferring immunity on an animal, including a human, comprises the step of administering to such animal a therapeutically effective immunization course at least one element of which is administering an immunization dose of a composition comprising an antigen and a multilamellar liposome comprising DMPC/cholesterol. In one aspect of the method the composition further comprises aluminum adjuvant such as aluminum hydroxide gel. In this method a particular liposome comprises a mole ratio of from about 80 to about 20 DMPC to from about 20 to about 80 cholesterol, preferably from about 70:30 to about 30:70 and most preferably about 70:30 particularly wherein the liposome is of equal solute distribution (e.g.,SPLV) and including the liposome being at least about 1 micron in diameter.

In the the composition for conferring immunity on an animal the antigen can be selected from the group comprising proteins, peptides, polysaccharides, bacterial fractions, viral fractions, protozoal fractions, synthetic peptides or lipopolysaccharides. The composition can further comprising an immunomodulator such as a cytokine. The composition can further comprise a suitable pharmaceutical carrier.

In a further aspect this invention includes a dosage form comprising an antigen and a liposome comprising DMPC/cholesterol 70:30 +/-5 (mole) in an immunization dose. The dosage form can further include aluminum adjuvant such as aluminum hydroxide gel. In some embodiment the liposome is a multilamellar such as an SPLV preferably of at least about 1 micron in diameter or a unilamellar liposome preferably at least about 1 micron in diameter. In embodiments of this dosage form including unilamellar liposomes the immunogen is selected from the group comprising proteins, peptides, polysaccharides, bacterial fractions, viral fractions, protozoal fractions, synthetic peptides or lipopolysaccharides and further an immunomodulator such as cytokine and a suitable pharmaceutical carrier.

In an additional aspect the invention includes a composition for potentiating an immune response in an animal, including a human, comprising an immunization dose of a composition comprising an immunogen and a liposome comprising DMPC/cholesterol 70:30 +/-5 (mole). The composition can further comprise aluminum adjuvant such as aluminum hydroxide gel. The liposomes of the composition include multilamellar liposomes such as SPLVs and unilamellar liposomes, and preferably wherein the liposomes are at least about 1 micron in diameter. In embodiments of this composition including unilamellar liposomes the antigen is selected from the group comprising proteins, peptides, polysaccharides, bacterial fractions, viral fractions, protozoal fractions, synthetic peptides or lipopolysaccharides and further an immunomodulator such as cytokine and a suitable pharmaceutical carrier.

In a further aspect the invention includes a composition for potentiating an immune response in an animal including as human comprising the use of an adjuvant wherein the adjuvant comprises a liposome comprising DMPC/cholesterol 70:30 +/-5 (mole). The composition can further comprise aluminum adjuvant such as aluminum hydroxide gel. The liposomes of the method include mutilamellar liposomes such as SPLVs and unilamellar liposomes, and preferably wherein the liposomes are at least about 1 micron in diameter.

Further entailed in this invention is a composition for priming an immune response in an animal, including a human, comprising a priming immunization dose of a composition comprising an adjuvant which is a liposome comprising DMPC/cholesterol 70:30 +/-5 (mole) and an adjuvant-obligatory immunogen such that administration of a booster dose of adjuvant-obligatory immunogen absent adjuvant potentiates immune response. The composition can further comprise an aluminum adjuvant such as aluminum hydroxide gel. The liposomes of the composition include multilamellar liposomes ouch as SPLVs and unilamellar liposomes, and preferably wherein the liposomes are at least about 1 micron in diameter.

A method of conferring immunity on an animal, including a human, comprises the step of administering to such animal a therapeutically effective immunization course at least one element of which is administering an immunization dose of a composition comprising an antigen and a liposome comprising DMPC/cholesterol 70:30 +/-5 (mole). The composition of the method can further comprise aluminum adjuvant such as aluminum hydroxide gel. The liposomes of the composition include multilamellar liposomes such as SPLVs and unilamellar liposomes, and preferably wherein the liposomes are at least about 1 micron in diameter. In embodiments of this composition including unilamellar liposomes the antigen is selected from the group comprising proteins, peptides, polysaccharides, bacterial fractions, viral fractions, protozoal fractions, synthetic peptides or lipopolysaccharides and further an immunomodulator such as a cytokine and a suitable pharmacautical carrier.

### Detailed Description of the Invention

It has now been discovered that such adjuvants of this invention comprise an influenza immunizing dosage form comprising a liposome and an antigen of Influenza wherein said liposome and antigen are present in an immunization dose. Such antigens of Influenza are the hemagglutinin fragment or the bromelain fragment.

In addition it has now been discovered that (1) salt forms of organic acid derivatives of sterols are particularly useful pharmaceutical adjuvants (and particularly in the form of liposomes and (ii) that DMPC/cholesterol liposomes (particularly multilamellar liposomes) are particularly useful pharmaceutical adjuvants (such adjuvants are advantageously used in aluminum hydroxide gels). Further preferred are DMPC/cholesterol liposomes, both multilamellar and unilamellar wherein the DMPC/cholesterol ratio is 70:30 +/-5 (mole).

The various terms used to define concepts in immunology are often loosely defined or otherwise misused. For clarity, in the discussion of this invention the following definitions will be used:
"Antigen" shall mean a substance or material that is recognized specifically by antibody and/or combines with an antibody.
"Adjuvant" shall mean a substance or material to potentiate an immune response when used in conjunction with an antigen. Adjuvants are further used to elicit immune response sooner, or a greater response, or with less antigen. Immunogen-obligatory adjuvant refers to an antigen which alone is not immunogenic but becomes immunogenic with adjuvant.
"Immunogen" shall mean a substance or material (including antigens) that is able to induce an immune response alone or in conjunction with an adjuvant. Both natural and synthetic substances may be immunogens. An immunogen will generally be a protein, peptide, polysaccharide, nucleoprotein, lipoprotein, synthetic polypeptide, or hapten linked to a protein, peptide, polysaccharide, nucleoprotein, lipoprotein or synthetic polypeptide or other bacterial, viral or protozoal fractions. It will be understood that "immunogen" includes substances which do not generate an immune response (or generate a only therapeutically ineffective immune response) unless associated with an adjuvant (e.g., small peptides) which will be referred to as "adjuvant-obligatory" immunogens.
"Immune response" shall mean a specific response of the immune system of an animal to antigen or immunogen. Immune responses may include the production of antibodies.
"Immunization conditions" shall mean factors which affect an immune response including the amount and kind of immunogen or adjuvant delivered to a subject animal including a human, method of delivery, number of inoculations, interval of inoculations, the type of subject animal and its condition.
"Vaccine" shall mean a pharmaceutical formulation able to induce immunity.
"Immunity" shall mean a state of resistance of a subject animal including a human to an infecting organism or substance. It will be understood that infecting organism or substance is defined broadly and includes parasites, toxic substances, cancers and cells as well as bacteria and viruses. A Therapeutically Effective Immunization Course will produce the immune response such as that exhibited by production of specific antibodies and/or reactivity of immune cells to antigen.
"Immunization dose" shall mean the amount of antigen or immunogen needed to precipitate an immune response. This amount will vary with the presence and effectiveness of various adjuvants.

This amount will vary with the animal and immunogen or antigen or adjuvant but will generally be between about 0.1ug/ml or less than about 500ug per inoculation. The immunization dose is easily determined by methods well known to those skilled in the art, such as by conducting statistically valid host animal immunization and challenge studies. See, for example, Manual or Clinical Immunology, H.R. Rose and H. Friedman, American Society for Microbiology, Washington, D.C. (1980). In some instances several immunization doses including booster doses will be administered to provide immunity, which collectively will be termed "Therapeutically Effective Immunization Course".

"Priming" shall mean the stimulation of a primary (as opposed to a secondary or later) response by a animal to a antigen. The primary response is characterized by the manufacture by the animal of antibody to the antigen, and ideally by the generation of a population of B-lymphocytes and T-lymphocytes that respond to secondary or later immunogenic challenge -- even absent adjuvant --with a rapid and substantive production of antibodies. Based upon such response 1, 2, 3 or more booster doses of immunogen, absent adjuvant, will generate a therapeutically effective immune response to the antigen.
In particular embodiments of this invention the liposomes will have a net charge or be neutral. Charged and particularly negatively charged liposomes may display superior adjuvancy to neutral liposomes.

A preferred class of lipids for forming liposomes are those of cholesterol hemisuccinate ("CHS"), such as those with sodium ("CHS_{sodium}") or tris(hydroxymethyl) aminomethane ("CHSₜᵣᵢₛ") as the counter ion, which are generally negatively charged.

Salt forms of an organic acid derivative of a sterol may be used in the practice of the invention. Generally any sterol which can be modified by the attachment of an organic acid may be used in the practice of the present invention. For example, such sterols include but are not limited to cholesterol, vitamin D, phytosterols (including but not limited to sitosterol, campesterol, stigmasterol, and the like), steroid hormones, and the like.

Organic acids which can be used to derivatize the sterols include but are not limited to the carboxylic acids, dicarboxylic acids, polycarboxylic acids, hydroxy acids, amino acids and polyamino acids. Because the salt forms increase the water solubility of organic acids, any organic acid may be used to derivatize the sterols; however an advantage may be obtained if the organic acid moiety itself is water soluble. Such water soluble organic acid moieties include but are not limited to water-soluble aliphatic carboxylic acids such acetic, propionic, butyric, valeric acids and the like (N.B., up to four-carbon acids are miscible with water; the five-carbon free acid is partly soluble and the longer chain free acids are virtually insoluble); water-soluble aliphatic dicarboxylic acids such as malonic, succinic, glutaric, adipic, pimelic, maleic and the like (N.B., the shorter chains are appreciably more soluble in water; borderline solubility in water occurs at C₆ to C₇); and water-insoluble aromatic dicarboxylic acids such as hemimellitic, trimesic, succinimide, and the like; polycarboxylic acids; water-soluble hydroxy acids such as glycolic, lactic, mandelic, glyceric, malic, tartaric, citric, and the like (N.B., alpha-hydroxy acids containing a branched chain attached to the alpha-carbon of the carbonyl group would be less susceptible to hydrolysis and, therefore, advantageous in the practice of the present invention); and any of the amino acids and polyamino acids. The organic acid can be linked to an hydroxyl group of the sterol via an ester or an ether bond using conventional methods (see, for example, U.S. Pat. Nos. 3,859,047; 4,040,784; 4,042,330; 4,183,847; and 4,189,400). The salt forms of the derivatized sterols can be prepared by dissolving both the organic acid derivative of the sterol and the counterion of the salt (e.g., the free base of the salt) in an appropriate volatile solvent, and removing the solvent by evaporation or a similar technique leaving a residue which consists of the salt form of the organic acid derivative of the sterol. Counterions that may be used include, but are not limited to, tris, 2-amino-2-methyl-1,3-propanediol, 2-aminoethanol, bis-tris propane, triethanolamine, and the like to form the corresponding salt. In fact, the free base of an ionizable bioactive agent such as miconazole free base and the like may be used as the counterion.

CHS forms liposomes when added to an aqueous material. This can conveniently be performed at 20°-25°C (room temperature) and atmospheric pressure. Agitation accelerates the process of liposome formation and is performed by such methods as vortexing, sonication or other methods well known in the art. If desired the resulting liposomes may be filtered or sized such as by passing through a filter stack such as a 0.4 or 0.2um filter (Nuclepore, Pleasanton, CA). Typically better adjuvant response is observed with greater amounts of lipid. Immunogens which partition into the liposome lamellae such as melanoma antigen in CHS liposomes may yield insufficient immunogenic responses without repeated inoculations and additional immuno stimulator. Without being bound by any particular theory it is believed that this partitioning results in the limitation of exposure of epitopes externally to the adjuvant liposomes. Immunogens may be modified by a number of methods well known in the art such as by amino acid addition or subtraction or conjugation with other moieties.

A preferred class of lipids for forming liposomes are those of dimyristoylphosphatidylcholine and cholesterol ("DMPC/cholesterol").

DMPC/cholesterol forms the required multilamellar liposomes over a wide range of proportion from about 100:1 (mole) to about 20:80. More preferred is about 70:30 to about 30:70, and yet further preferred is about 70:30. Additionally other lipids may be admixed with DMPC/cholesterol, such as dimyristoyl phosphatidylglycerol, dicetyl phosphate, phosphatidic acid, phosphatidylethanolamine, phosphatidylcholine and cholesterol hemisuccinate ("CHS"), such as those with sodium ("CHS_{sodium}") or tris(hydroxymethyl) aminomethane ("CHSₜᵣᵢₛ") as the counter ion.

Aluminum compounds are adjuvants well known in the art, and include aluminum hydroxide, aluminum phosphate, aluminum oxide or aluminum sulfate and will be termed collectively aluminum adjuvants By way of example, aluminum hydroxide is widely used in diphtheria and tetanus toxoid vaccines as well as in veterinary applications. Aluminum hydroxide powder spontaneously forms a gel upon hydration. To prepare a vaccine containing aluminum hydroxide, commonly immunogen in aqueous buffer is added to the preformed gel. Such vaccines are referred to as being aluminum-adsorbed.

DMPC/cholesterol multilamellar liposomes of the SPLV process are preferred but any other type of liposome may be used. The SPLV process generally involves. rotoevaporation of lipids in solvent in a round bottom flask to form a thin film. The lipid film is then dispersed in a non-water miscible solvent such as ether or methylene chloride to which the aqueous solute (containing immunogen) is then added. The mixture is then sonicated while being dried by a stream of nitrogen gas which drives off the organic solvent. The resultant liposome paste is resuspended in aqueous buffer. U.S. Patent No. 4,522,803 to Lenk, et al. further describes this process and is incorporated herein by reference. If desired the resulting liposomes may be filtered or sized such as by passing through a filter stack such as a 0.4 or 0.2um filter (Nuclepore, Pleasanton, CA).

The resulting liposomes are conveniently administered in aqueous material. The volume of aqueous material will vary with the particular liposome to be administered and is not critical. Generally about 0.5ml is a convenient liposome dosage volume. Typically better adjuvant response is observed with greater amounts of lipid.

Suitable aqueous material for either sterol or DMPC/cholesterol liposomes is saline solution, phosphate buffer or other well known aqueous pharmaceutical diluents.

These liposomes are conveniently associated with an immunogenic amount of antigen or immunogen. This association is engendered by mixing, adsorption, encapsulation, co-formation or other methods well known in the art.

The adjuvant effect of the instant invention is seen from the results in Tables 1, 2 and 3 as to sterol liposomes and Tables 4, 5, and 6 as to DMPC/cholesterol liposomes and Table 7 as to both sterol and DMPC/cholesterol liposomes. Table 1A compares the antibody response in guinea pigs immunized with Influenza B/Ann Arbor hemagglutinin (HA) alone or formulations with CHSₜᵣᵢₛ liposomes. HA elicits a particular level of antibody response without adjuvant, but this response is enhanced by adjuvant. CHSₜᵣᵢₛ liposomes are seen from the results to potentiate this antibody production. In particular, neutralizing antibody responses as detected by HAI to 5 or 0.5 ug of HA are increased up to about 50-fold and 70-fold respectively when HA is administered with the steroidal adjuvant.

Table 1B shows the antibody response in guinea pigs immunized with the bromelain fragment of HA (HAB) in various formulations. HAB is generally non-immunogenic when administered alone and is exemplary of an adjuvant-obligatory immunogen. Use of steroidal adjuvant in the form of CHS increases the immune response on a par or better than complete Freund's adjuvant, especially the production of protective neutralizing antibodies as detected by HAI. This increase may be about 1400-fold. Furthermore, increasing the amount of adjuvant increases the immune response.

Table 2 demonstrates the importance of priming in generation of the immune response. HAB is seen to generate a weak immune response that is not greatly potentiated on secondary challenge, even with adjuvant of this invention. However, when HAB is administered in association with the adjuvant of this invention, here in the form of a CHSₜᵣᵢₛ liposome a substantial immune response is generated upon application of a second inoculation of HAB, here in solution. It is an important aspect of this invention that priming of the subject animal with an immunogenic dose of adjuvant-obligatory immunogen and adjuvant permitted later booster doses to be highly effective wherein such booster doses did not contain adjuvant but were merely adjuvant-obligatory immunogen in solution. The priming of immune response with the adjuvant of this invention, permits booster administrations of adjuvant-obligatory immunogen without adjuvant, even when this immunogen would not have initially generated an immune response without coadministration with an adjuvant. In this embodiment of the invention it is important to note that any liposome can be used as a priming adjuvant, not just steroidal liposomes, for use with adjuvant-obligatory immunogens.

Table 3 discloses that superior results are obtained when the antigen is entrapped in an adjuvant liposome of the present invention and not merely mixed with the adjuvant liposome. This is particularly true when the priming response is considered. Table 3 In conjunction with Table 2 data indicates that entrapment is a potentiator of priming response, even more than a potentiator of secondary response. Table 3 shows also that changing the salt composition from tris to sodium does not alter the adjuvant effect provided by CHS liposomes.

Table 4 compares the antibody response in guinea pigs immunized with the bromelain fragment of Influenza B/Ann Arbor hemagglutinin (HAB) in various forms. HAB is an adjuvant-obligatory immunogen because it is poorly immunogenic when administered alone or when administered with aluminum hydroxide gel. The immunogenicity is greatly increased when HAB is entrapped within DMPC/cholesterol SPLV's at various mole ratios and lipid concentrations. Total anti-HA IgG responses detected by EIA are increased up to 5000 fold and protective neutralizing antibodies detected by HAI are increased up to 35 fold at 6 weeks when HAB is administered in DMPC/cholesterol SPLV's at 30:70 and 70:30 mole ratios, respectively. Increasing the lipid concentration also increases the adjuvant effect as demonstrated with 50:50 mole ratio formulations.

Table 5 demonstrates the adjuvant effect of DMPC/cholesterol SPLV's (70:30 mole ratio) on Influenza B/Ann Arbor hemagglutinin (HA). HA administered in free form is a relatively good immunogen at 5ug dose but elicits low antibody titers when administered at 0.5ug. Liposomal HA at both of these dosages generates responses which are up to 80 fold higher than free HA.

Table 6 demonstrates the adjuvant effect of DMPC/cholesterol SPLV's and aluminum hydroxide gel. As seen in Table 6 (Experiment 1), administration of HAB, with DMPC/cholesterol liposomes (200 mg starting lipid concentration) generates a strong anti-HA IgG response up to 100 fold greater and an HAI (neutralizing) antibody titer 10 fold greater than HAB alone. Increasing the starting lipid concentration to 500 mg increases the adjuvant effect even more (500 fold and 30 fold respectively). When the same formulations are administered with aluminum hydroxide gel, even these high titers are increased up to 3 times greater. The combined adjuvant effect for liposomes and aluminum hydroxide is clearly evident in (Table 6, Experiment II). As in the first experiment, liposomal HAB increases antibody responses up to 200 fold over free HAB (5ug dose). Administration with aluminum hydroxide gel substantially increases even these titers up to 5 times greater. Most striking is the observation that when a dose of 0.3ug liposomal HAB is administered with aluminum hydroxide a response equivalent to 5ug liposomal HAB is seen.

Table 7 shows the results obtained with HA used for vaccination after splitting the influenza virus with detergent. The results of Table 7 show that 5 mg HA in CHSₜᵣᵢₛ liposomes induced a strong adjuvant effect regardless whether of this mount of antigen was entrapped in 13.6 or 1.9 mg of lipid. The same observation can be made in the case of DMPC/chol formulations.

Furthermore, 1:10 dilution of CHSₜᵣᵢₛ formulation (0.5 ug HA) generated titers which were several fold higher than the non-entrapped antigen control at 0.5 or 5 ug HA.

Taken together the results showed that reduction of lipid content in formulation does not affect the adjuvant effect of liposomes.

Aluminum adjuvants are used in forms and proportions well known to those skilled in the art. Commercial preparations of aluminum hydroxide gel containing vaccines such as tetanus toxoids range from about 0.2 to about 1mg of aluminum/ml. The safe upper range is far higher for humans vaccines with as much as 15 mg or more of aluminum hydroxide per dose are known with no limit for veterinary applications.

Vaccines are conveniently administered in a dosage form. A "dosage form" will be understood to mean any pharmaceutically form of administering a vaccine including subcutaneous, oral, intramuscular, and ocular administration and utilizing vaccines in live, attenuated or synthetic or partial forms along with adjuvants and optionally immunomodulators such as cytokines. The combinations of the foregoing elements are prepared so that the dosage form is adapted to produce an immune response in the subject animal including a human as easily and effectively as possible.

The dosage forms including liposomal dosage forms resulting from the method of the present invention can be used therapeutically in animals such as mammals, including man, in the treatment of infections or conditions which require the delivery of immunogen in its bioactive form. Such conditions include but are not limited to disease states such as those that can be treated with vaccines. Extracorporeal treatment of immunoresponsive tissues is also contemplated.

Dosage forms also include micelle forms of the adjuvant as well as adjuvant incorporated into gel such as aluminum gels, liquid crystals, powders, precipitates and solutions. In particular embodiments the dosage form can be a unit dosage form configured and adapted to a single administration.

The mode of administration of the dosage form may determine the sites and cells in the organism to which the dosage form will be delivered. The dosage forms including liposomal dosage forms of the present invention can be administered alone but will generally be administered in admixture with a pharmaceutical carrier selected with regard to the intended route of administration and standard pharmaceutical practice. The dosage forms may be injected parenterally, for example, intra-muscularly or subcutaneously. The dosage forms may also be administered via the oral route. For parenteral administration, they can be used, for example, in the form of a sterile aqueous solution which may contain other solutes, for example, enough salts or glucose to make the solution isotonic. Other uses, depending upon the particular properties of the preparation, may be envisioned by those skilled in the art.

For administration to humans in the preventive or curative treatment of disease states responding to vaccine therapy, the prescribing physician will ultimately determine the appropriate therapeutically effective dosage for a given human subject, and this can be expected to vary according to the age, weight, and response of the individual as well as the nature and severity of the patient's disease. The dosage of the antigen in liposomal dosage form will generally be about that or less than that employed for the free antigen. In some cases, however, it may be necessary to administer dosages outside these limits.

### EXAMPLE 1

### Preparation of Adjuvant Liposomes with Antigen

50mg of CHSₜᵣᵢₛ (powdered) were placed into a 15ml test tube. 100ul of bromelain fragment of HA in aqueous buffer was added (673ug HAB, 0.01M phosphate buffered saline in 0.9%NaCl (wt.%)). The mixture was intermittently vortexed over a 2 hour period at 22.5°C+/-2.5°C and left until no large clumps were visible. The resultant liposomes were washed 3 times in 10ml of aqueous buffer solution being separated each time by 15 minutes of centrifugation (10,000rpm, J-20 rotor (Beckman, Palo Alto, CA)). The final pellet was brought to 4.0ml in buffer and sealed in an amber vial under nitrogen.

### EXAMPLE 2

### Preparation of Adjuvant Liposomes with Antigen

500mg of CHSₜᵣᵢₛ (powdered) were placed into a 15ml test tube. 1ml of HA in aqueous buffer was added (300ug HA, 0.01M phosphate buffered saline in 0.9%NaCl) The mixture was intermittently vortexed over a 2 hour period at 22.5°C+/-2.5°C. The resultant liposomes were washed 3 times in 10ml of aqueous buffer solution being separated each time by 15 minutes of centrifugation (10,000rpm, J-20 rotor (Beckman, Palo Alto, CA)). The final pellet was brought to 4.0ml in buffer and sealed in an amber vial under nitrogen.

### EXAMPLE 3

### Preparation of Adjuvant Liposomes with Antigen

500mg of CHSₜᵣᵢₛ (powdered) were placed into a 15ml test tube. 1ml of bromelain fragment of HA in aqueous buffer was added (673ug HAB, 0.01M phosphate buffered saline in 0.9%NaCl) The mixture was intermittently vortexed over a 2 hour period at 22.5°C+/-2.5°C. The resultant liposomes were washed 3 times in 10ml of aqueous buffer solution being separated each time by 15 minutes of centrifugation (10,000rpm, J-20 rotor). The final pellet was brought to 4.0ml in buffer and sealed in an amber vial under nitrogen.

### EXAMPLE 4

### Preparation of Adjuvant Liposomes with Antigen

500mg of CHSₜᵣᵢₛ (powdered) were placed into a 15ml test tube. 2ml of bromelain fragment of HA in aqueous buffer was added (1,100ug HAB, 0.9%NaCl) The mixture was intermittently vortexed over a 2 hour period at 22.5°C+/-2.5°C. The resultant liposomes were washed 3 times in 10ml of 0.9%NaCl solution being separated each time by 15 minutes of centrifugation (10,000rpm, J-20 rotor). The final pellet was brought to 4.0ml in buffer and sealed in an amber vial under nitrogen.

### EXAMPLE 5

### Preparation of Adjuvant Liposomes with Antigen

1500mg of CHSₜᵣᵢₛ (powdered) were placed into a 15ml test tube. 3ml of bromelain fragment of HA in aqueous buffer was added (673ug HAB, 0.01M phosphate buffered saline in 0.9%NaCl) The mixture was intermittently vortexed over a 2 hour period at 22.5°C+/-2.5°C. The resultant liposomes were washed 3 times in 10ml of aqueous buffer solution being separated each time by 15 minutes of centrifugation (10,000rpm, J-20 rotor). The final pellet was brought to 4.0ml in buffer and sealed in an amber vial under nitrogen.

### EXAMPLE 6

### Preparation of Adjuvant Liposomes with Antigen

500mg of CHS_{sodium} (powdered) were placed into a 15ml test tube. 1ml of bromelain fragment of HA in aqueous buffer was added (673ug HAB, 0.01M phosphate buffered saline in 0.9%NaCl) The mixture was intermittently vortexed over a 2 hour period at 22.5°C+/-2.5°C. The resultant liposomes were washed 3 times in 10ml of aqueous buffer solution being separated each time by 15 minutes of centrifugation (10,000rpm, J-20 rotor). The final pellet was brought to 4.0ml in buffer and sealed in an amber vial under nitrogen.

### EXAMPLE 7

### Adjuvancy Exhibited

Liposomes containing HAB were prepared as in Example 2. Entrapment values were determined by SRID and the liposomes were diluted in saline to a concentration of 10 ug protein/ml and were sealed in a glass vial with a rubber stopper and crimp seal.

Five 450-500g male Hartley guinea pigs (Buckberg Lab Animals, Landis Store, PA) were injected intramuscularly with 0.5 ml of the liposome suspension or with 0.5 ml of free HAB in the right hind leg (5 ug). At 4 weeks post immunization, the guinea pigs were lightly anesthetized with ether and approximately 4 ml of blood was drawn by cardiac puncture. The blood was allowed to clot at room temperature overnight. The blood was centrifuged, and the serum was drawn off and stored at 4°C until tested. The day after bleeding, the guinea pigs were again injected i.m. with 0.5 ml of free or liposomal HAB (5 ug), this time in the left hind leg. Blood was collected in the same manner to one year after the initial injection.

Total anti-HA IgG antibodies in the serum samples were determined by Enzyme Immunoassay (EIA) and neutralizing antibodies were determined by Hemagglutination Inhibition Assay (HAI). The results are shown in Tables 1, 2 and 3.

### EXAMPLE 8

### Preparation of Adjuvant Liposomes with Antigen

100mg of cholesterol and 400mg of DMPC were placed into a 500ml round bottom flask and suspended in 3ml chloroform and dried to a film by rotoevaporation. 20ml of anhydrous ether was added to the flask followed by 1.5ml of HA in aqueous buffer was added (915ug HA, 0.01M phosphate buffered saline in 0.9%NaCl) -- "aqueous buffer"). The mixture was covered loosely with foil and sonicated in a 40°C water bath while concurrently evaporating the ether with a gentle stream of nitrogen gas. The resultant lipid paste was thoroughly dried under nitrogen until no trace of ether was noted by smell. 10 ml of buffer was added to the flask and the liposome suspension was transferred to a 15ml test tube. The resultant liposomes were washed 3 times in 10ml of aqueous buffer being separated each time by 10 minutes of centrifugation (10,000rpm, J-20 rotor (Beckman, Palo Alto, CA)). The final pellet was brought to 6.0ml in buffer and sealed in an amber vial under nitrogen.

### EXAMPLE 9

### Preparation of Adjuvant Liposomes with Antigen

100mg of cholesterol and 400mg of DMPC were placed into a 500ml round bottom flask and suspended in 3ml chloroform and dried to a film by rotoevaporation. 20ml of anhydrous ether was added to the flask followed by 1.5ml of HAB in aqueous buffer was added (1,000ug HAB, 0.01M phosphate buffered saline in 0.9%NaCl). The mixture was covered loosely with foil and sonicated in a 40°C water bath while concurrently evaporating the ether with a gentle stream of nitrogen gas. The resultant lipid paste was thoroughly dried under nitrogen until no trace of ether was noted by smell. 10 ml of aqueous buffer was added to the flask and the liposome suspension was transferred to a 15ml test tube. The resultant liposomes were washed 3 times in 10ml of aqueous buffer solution being separated each time by 10 minutes of centrifugation (10,000rpm, J-20 rotor (Beckman, Palo Alto, CA)). The final pellet was brought to 6.0ml in buffer and sealed in an amber vial under nitrogen.

### EXAMPLE 10

### Preparation of Adjuvant Liposomes with Antigen

92mg of cholesterol and 108mg of DMPC were placed into a 100ml round bottom flask and suspended in 3ml chloroform and dried to a film by rotoevaporation. 10ml of anhydrous ether was added to the flask followed by 2.0ml of HAB in aqueous buffer was added (1,100ug HAB, 0.01M phosphate buffered saline in 0.9%NaCl). The mixture was covered loosely with foil and sonicated in a 40°C water bath while concurrently evaporating the ether with a gentle stream of nitrogen gas. The resultant lipid paste was thoroughly dried under nitrogen until no trace of ether was noted by smell. 10 ml of buffer was added to the flask and the liposome suspension was transferred to a 15ml test tube. The resultant liposomes were washed 3 times in 10ml of aqueous buffer solution being separated each time by 10 minutes of centrifugation (10,000rpm, J-20 rotor (Beckman, Palo Alto, CA)). The final pellet was brought to 6.0ml in buffer and sealed in an amber vial under nitrogen.

### EXAMPLE 11

### Preparation of Adjuvant Liposomes with Antigen

40mg of cholesterol and 160mg of DMPC were placed into a 100ml round bottom flask and suspended in 3ml chloroform and dried to a film by rotoevaporation. 10ml of anhydrous ether was added to the flask followed by 2.0ml of HAB in aqueous buffer was added (1,100ug HAB, 0.01M phosphate buffered saline in 0.9%NaCl). The mixture was covered loosely with foil and sonicated in a 40°C water bath while concurrently evaporating the ether with a gentle stream of nitrogen gas. The resultant lipid paste was thoroughly dried under nitrogen until no trace of ether was noted by smell. 10 ml of buffer was added to the flask and the liposome suspension was transferred to a 15ml test tube. The resultant liposomes were washed 3 times in 10ml of aqueous buffer solution being separated each time by 10 minutes of centrifugation (10,000rpm, J-20 rotor (Beckman, Palo Alto, CA)). The final pellet was brought to 6.0ml in buffer and sealed in an amber vial under nitrogen.

### Example 12

### Preparation of Gel Admixed with Liposomes

Aluminum hydroxide gel, 2% (Alhydrogel™; Connaught Laboratories, Inc., Swiftwater, PA) containing 7.29mg/ml aluminum was used in conjunction with 1.02ml of the liposomes of this invention prepared as in Example 10. The liposomes were admixed with 0.67ml aluminum hydroxide gel and 5.31ml of saline. The final aluminum concentration was 0.7mg/ml and the HAB concentration was 10ug/ml. The mixture was sealed in a glass vial with rubber stopper and crimp seal.

### EXAMPLE 13

### Adjuvancy

Liposomes containing HAB were prepared as in Example 9. Entrapment values were determined by SRID and the liposomes were diluted in saline to a concentration of 10 ug protein/ml and were sealed in a glass vial with a rubber stopper and crimp seal.

Five 450-500g male Hartley guinea pigs (Buckberg Lab Animals, Landis Store, PA) were injected intramuscularly with 0.5 ml of the liposome suspension or with 0.5 ml of the liposome suspension or with 0.5 ml of free HAB in the right hind leg (5ug). At 4 weeks post immunization, the guinea pigs were lightly anesthetized with ether and approximately 4 ml of blood was drawn by cardiac puncture. The blood was allowed to clot at room temperature overnight. The blood was centrifuged, and the serum was drawn off and stored at 4°C until tested. The day after bleeding, the guinea pigs were again injected i.m. with 0.5 ml of free or liposomal HAB (5ug), this time in the left hind leg. Blood was collected in the same manner up to one year after the initial injection.

Total anti-HA IgG antibodies in the serum samples were determined by Enzyme Immunoassay (EIA) and neutralizing antibodies were determined by Hemagglutination Inhibition Assay (HAI). The results are shown in Tables 4, 5 and 6.

### Example 14

### Melanoma Procedures

Liposomes containing ganglioside GD₃ (kindly provided by Dr. P Livingston, Sloan Kettering Institute for Cancer Research, New York, New York) were prepared at 10:1 and 100:1 (w/w) lipid to antigen (GD₃) ratio using 10mg lipid to 1 mg GD₃ and 150mg lipid to 1.5 mg GD₃. Liposomes were prepared using either CHSₜᵣᵢₛ or DMPC/cholesterol (70:30 mole percent).

GD₃ antigen suspended in phosphate buffered saline (PBS but without Ca⁺⁺ or Mg⁺⁺) at pH 7.2 was entrapped in either CHSₜᵣᵢₛ lipid liposomes or DMPC/cholesterol (70:30 mole percent) lipid liposomes. CHSₜᵣᵢₛ/GD₃ MLV liposomes were made by Method A (below) and DMPC/cholesterol/GD₃ SPLV liposomes by Method B (below).

Mice ware injected 4 times with the liposomal antigen preparation and antibody level tested. Antibody to melanoma GD₃ was produced. It was noted however that other melanoma vaccine preparations yielded higher antibody titers in mice than the instant preparation by the regimen described herein.

### Method A

10 mg CHSₜᵣᵢₛ (powdered) and 1 mg GD₃ (10:1 ratio) (wt/wt) in 50 ul PBS were placed in a test tube and hydration of lipids allowed for 2 hours at room temperature with intermittent vortexing. The lipidic material was resuspended in 10 ml PBS without Ca⁺⁺ or Mg⁺⁺ and centrifuged at 13,000 rpm for 30 minutes, two times. The final liposome pellet was resuspended in 2.5 ml PBS. The liposome preparation was sealed in an amber vial under nitrogen until testing. An additional formulation at 100:1 lipid/antigen ratio was prepared as above, using 150 mg CHSₜᵣᵢₛ and 1.5 mg GD₃ in 300 ul PBS.

### Method B

2 mg cholesterol and 8 mg DMPC in 0.5 ml chloroform were dried by rotoevaporation in a 25 ml round-bottom flask, resolubilized in 2 ml anhydrous ether and mixed with 1.5 mg GD₃ in 60 ul PBS. The resulting mixture was sonicated at 40°C under a stream of N₂ to form a lipid paste which was further dried under N₂. The dry material was resuspended in 10 ml PBS and centrifuged at 10,000 rpm for 10 minutes, three times. The final liposome pellet was brought to 2.28 at and supplemented with Alhydrogel (0.7 mg Al/ml). An additional formulation at 100:1 lipid/antigen ratio was prepared as above using 120 mg DMPC, 30 mg cholesterol, and 1.5 mg GD₃ in 0.45 ml PBS.

### Example 15

### Additional Immune Response Studies

To determine further the role of the dose of both HA (split antigen) and lipid, guinea-pigs were inoculated with liposome formulations at 5 or 0.5 ug HA each entrapped in various amount of lipid (Table 7).

Two CHSₜᵣᵢₛ formulations were prepared as described in Example 2 to give a dose of 5.0 by HA and either 13.6 mg lipid (MLV, CHSₜᵣᵢₛ, H) or 1.9 mg lipid as multilamellae vesicles of CHS (MLV, CHSₜᵣᵢₛ, L). The "H" and "L" designations refer to high and low amount of lipid in particular dosages. The demarcation of high and low is arbitrary but convenient in conceptualizing any role that the relative amount of lipid plays in vaccine efficacy.) Two DMPC/CHOL formulations were prepared as described in Example 8 to give a dose of 5.0 mg HA and either 9.7 mg lipid of stable plurilamellar vesicles, dimyristoylphosphatidylcholine/cholesterol (SPLV, DMPC/C, H) or 2.9 mg lipid (SPLV, DMPC/C, L).
In addition each H formulation exhibiting a relatively high amount of lipid per 0.5 ml inoculum was further diluted 1:10 in PBS and the resulting suspensions containing 0.5 ug HA and either 1.4 mg CHSₜᵣᵢₛ or 1.0 mg DMPC/CHOL also inoculated in the same vial. Inoculation of guinea pigs and determination of antibody by EIA or HAI were performed as described in Example 7 and Example 15.

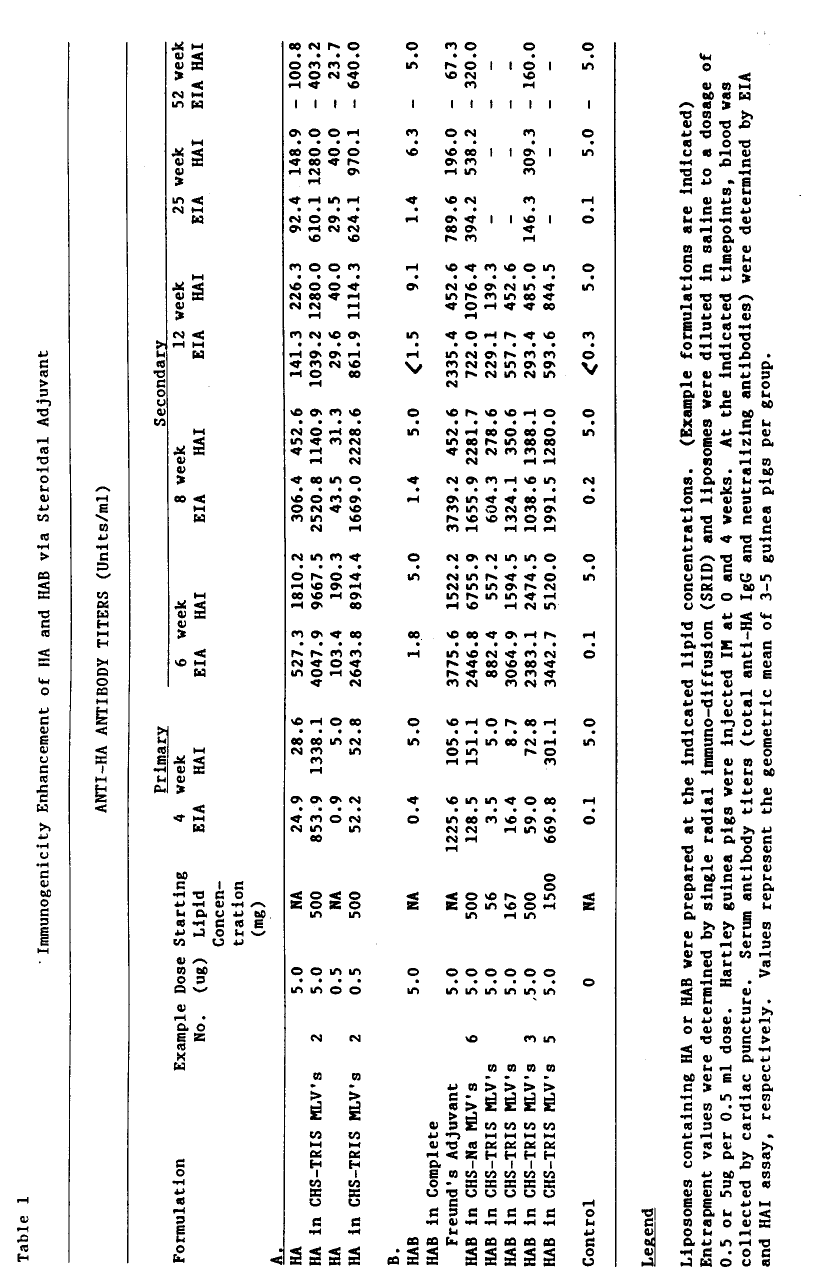

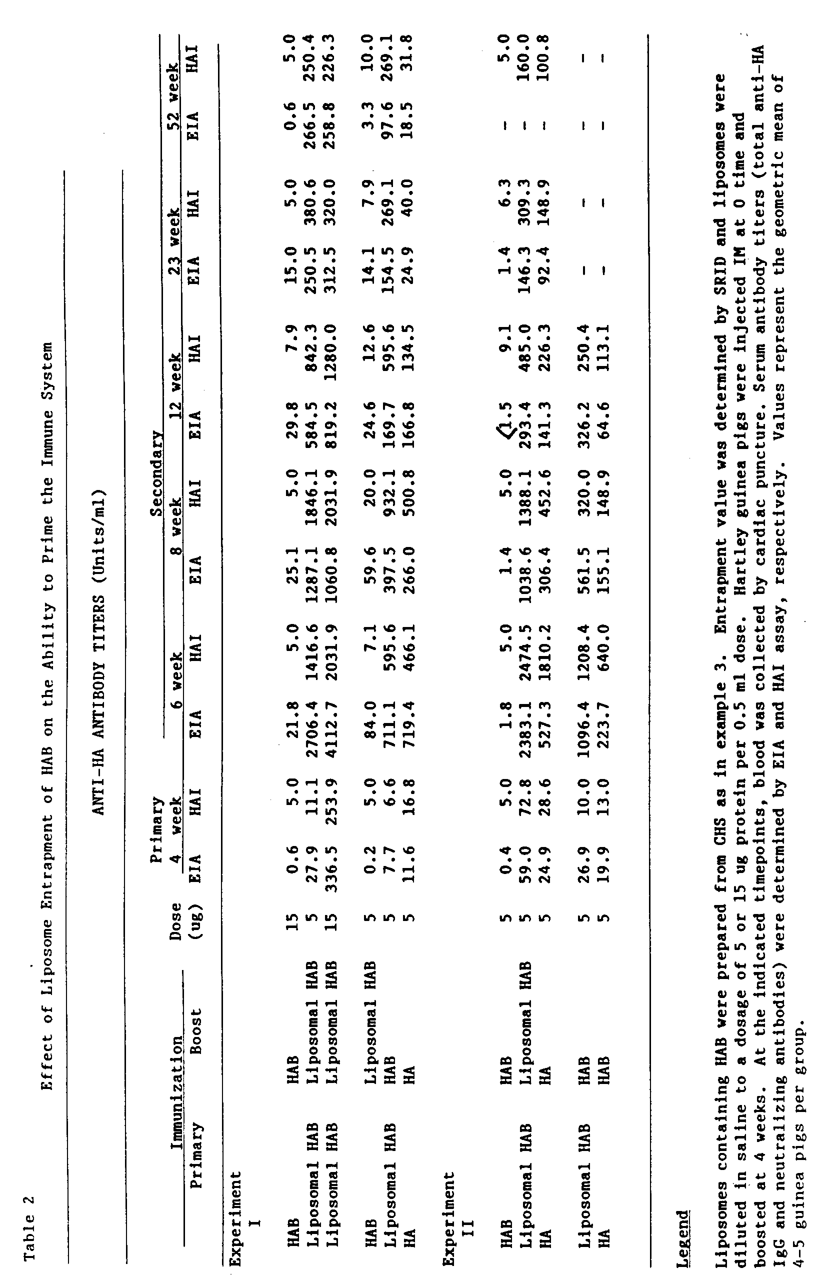

**Table 3**

| Steroidal adjuvant effect after mixing or entrapping HAB ANTI-HA antibody titers (Units/ml) | | | | | | | | |
|---|---|---|---|---|---|---|---|---|
| Formulation | Primary | | Secondary | | | | | |
| | 4 week | | 6 week | | 8 week | | 12 week | |
| | EIA | HAI | EIA | HAI | EIA | HAI | EIA | HAI |
| HA | 29.6 | 23.4 | 683.6 | 556.3 | 370.3 | 294.8 | 173.8 | 254.0 |
| HAB | 0.2 | 5.0 | 0.6 | 5.0 | 0.8 | 5.0 | 0.7 | 5.0 |
| HAB in CHS-Tris MLV's | 49.8 | 57.7 | 1998.3 | 842.3 | 1188.5 | 640.0 | - | - |
| HAB + CHS-Tris MLV's | 1.6 | 5.7 | 731.2 | 263.0 | 425.8 | 105.3 | - | - |
| HAB in CHS-Na MLV's | 34.1 | 37.6 | 2213.2 | 1280.0 | 799.6 | 604.2 | 39.7 | 485.0 |
| HAB + CHS-Na MLV's | 2.0 | 5.0 | 857.0 | 121.3 | 483.6 | 54.0 | - | - |
| Legend: HAB was entrapped in liposomes or mixed with empty liposomes. All liposomes were prepared using 500 mg of lipid. The inoculum contained 5 ug HAB or HA/0.5 ml. The experiment was conducted as described in the legend of Table 1. | | | | | | | | |

**Table 4**

| Enhancement of Immunogenicity of HAB by use of DMPC:cholesterol SPLV's at various mole ratios and lipid concentrations | | | | | | | | |
|---|---|---|---|---|---|---|---|---|
| Formulation | DMPC: Cholesterol mole ratio | Starting Lipid concentration (mg) | Primary | | Secondary | | | |
| | | | 4 week | | 6 week | | 8 week | |
| | | | EIA | HAI | EIA | HAI | EIA | HAI |
| HAB | NA | NA | 0.1 | 5.0 | 0.1 | 5.0 | 0.1 | 6.0 |
| HAB in Aluminum hydroxide gel | NA | NA | 0.1 | 5.0 | 1.8 | 5.0 | 8.0 | 5.0 |
| Liposomal HAB | 70:30 | 200 | 16.4 | 64.5 | 425.1 | 177.1 | 161.0 | 125.2 |
| | 50:50 | 50 | 7.1 | 4.0 | 171.0 | 5.0 | 119.3 | 5.0 |
| | 50:50 | 200 | 0.7 | 6.0 | 63.9 | 34.8 | 43.7 | 18.9 |
| | 50:50 | 500 | 31.1 | 23.4 | 200.1 | 47.6 | 89.0 | 40.0 |
| | 40:60 | 200 | 11.0 | 11.0 | 465.5 | 80.0 | 167.2 | 82.4 |
| | 30.70 | 200 | 18.7 | 5.0 | 492.2 | 56.6 | 259.9 | 36.6 |
| Control | NA | 0 | 0.1 | 5.5 | 0.1 | 5.0 | 0.1 | 5.0 |
| Legend: DMPC:Cholesterol SPLV's containing HAB were prepared at the indicated lipid concentrations and mole ratios. Entrapment values were determined by SRID and liposomes were diluted in saline to a dosage of 5ug per 0.5 ml dose. Hartley guinea pigs were injected i.m. at 0 and 4 weeks. At the indicated timepoints, blood was collected by cardiac puncture. Serum antibody titers (total anti-HA IgG and neutralizing antibodies) were determined by EIA and HAI assay, respectively. Values represent the geometric mean of 4-5 guinea pigs per group. | | | | | | | | |

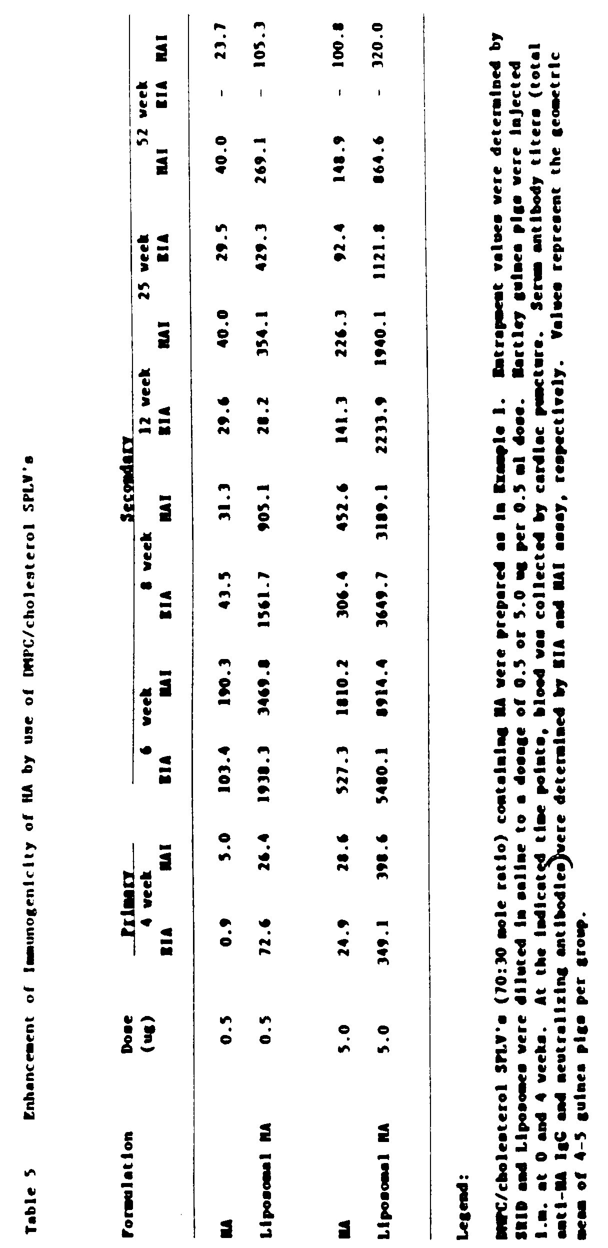

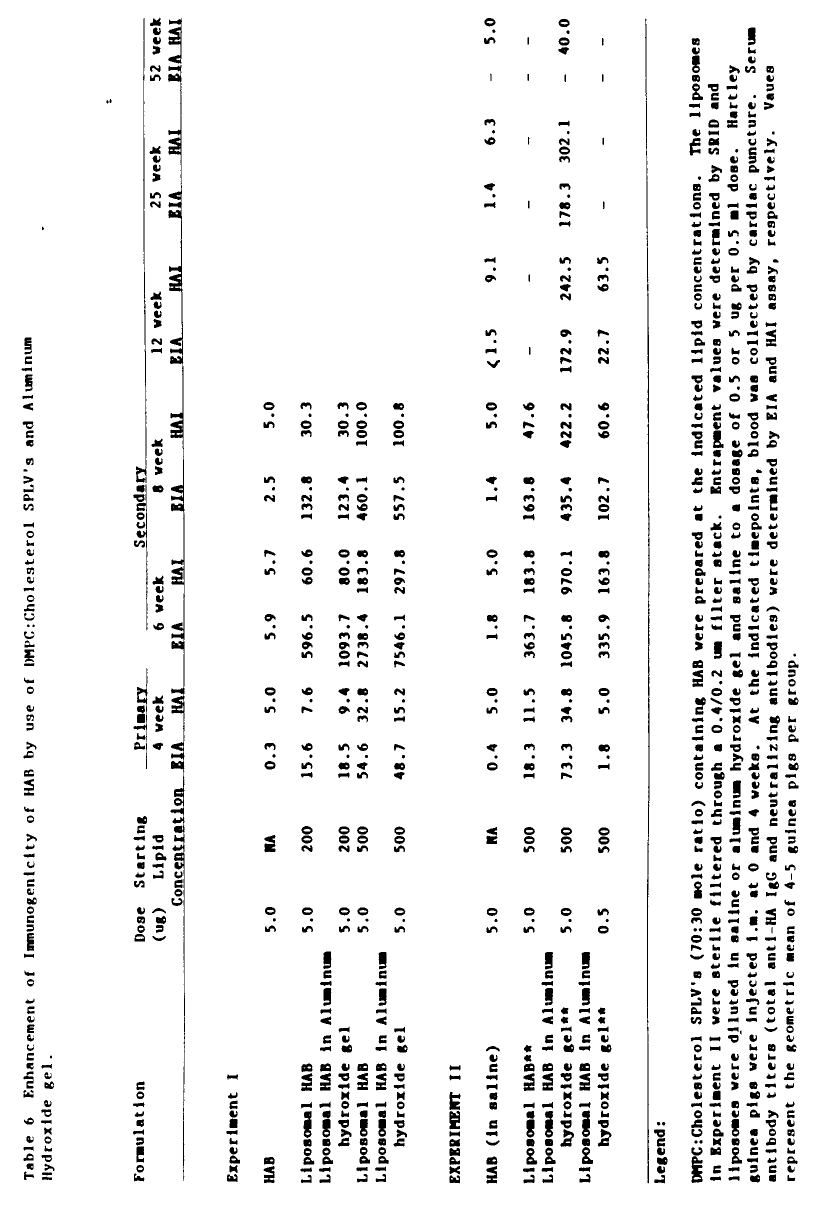

**Table 7**

| ANTI-HA ANTIBODIES IN SERA OF MICE IMMUNIZED WITH HA ENTRAPPED IN LIPOSOMES | | | | | | | | | | |
|---|---|---|---|---|---|---|---|---|---|---|
| | DOSE/0.5 ml/INOCULUM | | 4 week | | 6 week | | 8 week | | 12 week | |
| HA FORMULATION | PROTEIN(ug), | LIPID(mg) | EIA | HAI | EIA | HAI | EIA | HAI | EIA | HAI |
| Non-Entrapped | 5.0 | - | 26 | 29 | 516 | 557 | 301 | 368 | 198 | 320 |
| | 0.5 | - | 3 | 9 | 205 | 243 | 126 | 211 | 57 | 106 |
| MLV, CHST, H | 5.0 | 13.6 | 891 | 761 | 7062 | 7241 | 2170 | 3044 | 1581 | 1318 |
| | 0.5 | 1.4 | 52 | 33 | 2655 | 2941 | 1018 | 1237 | 557 | 640 |
| MLV, CHST, L | 5.0 | 1.9 | 147 | 92 | 3886 | 5553 | 3055 | 1810 | 1166 | 1191 |
| | - | - | - | - | - | - | - | - | - | - |
| SPLV, DMPC/C, H | 5.0 | 9.7 | 123 | 80 | 2204 | 1940 | 1548 | 1470 | 776 | 640 |
| | 0.5 | 1.0 | 22 | 23 | 387 | 399 | 233 | 243 | 136 | 121 |
| SPLV, DMPC/C, L | 5.0 | 2.9 | 101 | 89 | 1766 | 1810 | 1042 | 1613 | 767 | 905 |
| | - | - | - | - | - | - | - | - | - | - |
| A/ Liposomes (MLV, SPLV) were prepared with either 500 mg (H) or 50 mg (L) of lipid (CHST or DMPC/C, 70:30 mole percent) B/ Geometric mean titer (n-51) determined by enzyme-linked immuno assay (EIA) and hemagglutination imhibition (HAI) | | | | | | | | | | |

## Claims

1. A dosage form comprising an antigen and a liposome comprising a salt form of an organic acid derivative of a sterol as an adjuvant, wherein said antigen and liposome are present in an immunization dose.

2. The dosage form of claim 1 wherein said salt form comprises a tris (hydroxymethyl)aminomethane or a sodium salt form.

3. The dosage form of any one of claims 1 or 2 wherein said organic acid comprises a carboxylic acid, a dicarboxylic acid, or a polycarboxylic acid.

4. The dosage form of claim 3 in which the organic acid comprises an aliphatic carboxylic acid containing up to five carbon atoms.

5. The dosage form of claim 3 in which the organic acid comprises an aliphatic dicarboxylic acid containing up to seven carbon atoms.

6. The dosage form of claim 5 in which the aliphatic decarboxylic acid derivative is a succinate.

7. The dosage form of any of claims 1 to 6, wherein the liposome comprises a salt form of cholesterol hemisuccinate.

8. The dosage form of claim 7, wherein the liposome comprises a tris (hydroxymethyl) aminomethane salt form of cholesterol hemisuccinate.

9. The dosage form of any one of claims 1 to 8 wherein said liposome is a multilamellar vesicle.

10. The dosage form of any one of claims 1 to 8 wherein said liposome is at least 1 micron in diameter.

11. The dosage form of any one of claims 1 to 10 wherein the antigen is selected from the group comprising proteins, peptides, polysaccharides, nucleic acids, lipids, glycolipids, lipoproteins, lipopolysaccharides, synthetic peptides or bacterial, viral, protozoal, tissue, or cellular fractions.

12. The dosage form of claim 11 wherein said antigen is an influenza antigen.

13. The dosage form of claim 12 wherein said influenza antigen comprises hemagglutinin or a fragment thereof.

14. The dosage form of claim 13 wherein said antigen comprises bromelain fragment of hemagglutinin.

15. The dosage form of claim 12 wherein said influenza antigen comprises the bromelain fraction.

16. The dosage form of claim 12 wherein said influenza antigen comprises neuraminidase.

17. The dosage form of any one of claims 1 to 16 further comprising an immunomodulator.

18. The dosage form of claim 17 wherein the immunomodulator is a cytokine.

19. The dosage form of any one of claims 1 to 18 wherein the antigen is entrapped in the liposome.

20. The dosage form of any one of claims 1 to 19 further comprising a suitable pharmaceutical carrier.

21. A composition for potentiating or priming an immune response in an animal, including a human, comprising an immunization dose of a dosage form of any one of claims 1 to 20 and a pharmaceutical vehicle.

## Patentansprüche

1. Dosierungsform, umfassend ein Antigen und ein Liposom, umfassend eine Salzform eines organischen Säurederivats eines Sterols als ein Adjuvans, worin das Antigen und das Liposom in einer Immunisierungsdosis vorliegen.

2. Dosierungsform nach Anspruch 1, worin die Salzform die Form eines Tris(hydroxymethyl)aminomethan- oder Natriumsalzes umfaßt.

3. Dosierungsform nach einem der Ansprüche 1 oder 2, worin die organische Säure eine Carbonsäure, eine Dicarbonsäure oder eine Polycarbonsäure umfaßt.

4. Dosierungsform nach Anspruch 3, worin die organische Säure eine aliphatische Carbonsäure mit bis zu 5 Kohlenstoffatomen umfaßt.

5. Dosierungsform nach Anspruch 3, worin die organische Säure eine aliphatische Dicarbonsäure mit bis zu 7 Kohlenstoffatomen umfaßt.

6. Dosierungsform nach Anspruch 5, worin das aliphatische Dicarbonsäurederivat ein Succinat ist.

7. Dosierungsform nach einem der Ansprüche 1 bis 6, worin das Liposom eine Salzform von Cholesterin-hemisuccinat umfaßt.

8. Dosierungsform nach Anspruch 7, worin das Liposom eine Tris(hydroxymethyl) aminomethan-Salzform von Cholesterin-hemisuccinat umfaßt.

9. Dosierungsform nach einem der Ansprüche 1 bis 8, worin das Liposom ein multilamellares Vesicel ist.

10. Dosierungsform nach einem der Ansprüche 1 bis 8, worin das Liposom einen Durchmesser von mindestens 1 Mikron hat.

11. Dosierungsform nach einem der Ansprüche 1 bis 10, worin das Antigen ausgewählt ist aus der Gruppe von Proteinen, Peptiden, Polysacchariden, Nucleinsäuren, Lipiden, Glycolipiden, Lipoproteinen, Lipopolysacchariden, synthetischen Peptiden oder bakteriellen, viralen, protozoalen Geweben oder Zellfraktionen.

12. Dosierungsform nach Anspruch 11, worin das Antigen ein Influenza-Antigen ist.

13. Dosierungsform nach Anspruch 12, worin das Influenza-Antigen Hämagglutinin oder ein Fragment davon ist.

14. Dosierungsform nach Anspruch 13, worin das Antigen ein Bromelain- Fragment von Hämmagglutinin umfaßt.

15. Dosierungsform nach Anspruch 12, worin das Influenza-Antigen die Bromelain-Fraktion umfaßt.

16. Dosierungsform nach Anspruch 12, worin das Influenza-Antigen Neuramindase umfaßt.

17. Dosierungsform nach einem der Ansprüche 1 bis 16, die außerdem einen Immunmodulator umfaßt.

18. Dosierungsform nach Anspruch 17, worin der Immunmodulator ein Cytokin ist.

19. Dosierungsform nach einem der Ansprüche 1 bis 18, worin das Antigen in das Liposom eingeschlossen ist.

20. Dosierungsform nach einem der Ansprüche 1 bis 19, die außerdem einen geeigneten pharmazeutischen Träger umfaßt.

21. Zusammensetzung zur Potenzierung oder Auslösung einer Immunantwort in einem Tier, einschließlich eines Menschen, das eine Immunisierungsdosis einer Dosisform nach einem der Ansprüche 1 bis 20 mit einem pharmazeutischen Vehiculum umfaßt.

## Revendications

1. Une forme de dosage comprenant un antigène et un liposome comportant une forme de sel dérivé d'acide organique d'un stérol, en tant qu'adjuvant, dans laquelle ledit antigène et ledit liposome sont présents dans une dose d'immunisation.

2. La forme de dosage de la revendication 1, dans laquelle ladite forme de sel comporte un tris (hydroxyméthyle) aminométhane ou une forme de sel de sodium.

3. La forme de dosage de l'une quelconque des revendications 1 ou 2, dans laquelle ledit acide organique comprend un acide carboxylique, un acide dicarboxylique ou un acide polycarboxylique.

4. La forme de dosage selon la revendication 3, dans laquelle l'acide organique comprend un acide aliphatique carboxylique. contenant jusqu'à 5 atomes de carbone.

5. La forme de dosage de la revendication 3, dans laquelle l'acide organique comprend un acide aliphatique dicarboxylique renfermant jusqu'à 7 atomes de carbone.

6. La forme de dosage de la revendication 5, dans laquelle le dérivé d'acide aliphatique dicarboxylique est un succinate.

7. La forme de dosage selon l'une quelconque des revendications 1 à 6, dans laquelle le liposome comprend une forme de sel d'hémisuccinate de cholestérol.

8. La forme de dosage selon la revendication 7, dans laquelle le liposome comporte une forme de sel de tris (hydroxyméthyle) aminométhane d'hémisuccinate de cholestérol.

9. La forme de dosage de l'une quelconque des revendications 1 à 8, dans laquelle le liposome est une vésicule plurilamellaire.

10. La forme de dosage de l'une quelconque des revendications 1 à 8, dans laquelle ledit liposome présente un diamètre d'au moins 1 micron.

11. La forme de dosage de l'une quelconque des revendications 1 à 10, dans laquelle l'antigène est choisi dans le groupe comportant des protéines, des peptides, des polysaccharides, des acides nucléiques, des lipides, des glycolipides, des lipoprotéines, des lipopolysaccharides, des peptides synthétiques ou des fractions bactérienne, virale, protozoaire, de tissu ou cellulaires.

12. La forme de dosage de la revendication 11 , dans laquelle ledit antigène est un antigène de la grippe.

13. La forme de dosage de la revendication 12, dans laquelle ledit antigène de la grippe comprend de l'hémagglutinine ou un fragment de celle-ci.

14. La forme de dosage de la revendication 13, dans laquelle ledit antigène comprend un fragment de bromélaïne d'hémagglutinine.

15. La forme de dosage de la revendication 12, dans laquelle ledit antigène de la grippe comprend la fraction bromélaïne

16. La forme de dosage de la revendication 12, dans laquelle ledit antigène de la grippe comprend de la neuramidinase.

17. La forme de dosage de l'une quelconque des revendications 1 à 16, comportant en outre un immunomodulateur.

18. La forme de dosage de la revendication 17, dans laquelle l'immunomodulateur est une cytokine.

19. La forme de dosage de l'une quelconque des revendications 1 à 18, dans laquelle l'antigène est piégé dans le liposome.

20. La forme de dosage de l'une quelconque des revendications 1 à 19, comportant en outre un support pharmaceutique approprié.

21. Une composition pour potentialiser ou favoriser une réponse immunitaire chez un animal, y compris un être humain comportant une dose d'immunisation d'une l'orme de dosage selon l'une quelconque des revendications 1 à 20 et un véhicule pharmaceutique.
